# EUROPÄISCHE PATENTSCHRIFT

(11) **EP 0 348 793 B1**
(45) Veröffentlichungstag und Bekanntmachung des Hinweises auf die Patenterteilung: **22.09.1993**
(21) Anmeldenummer: 89111192.4
(22) Anmeldetag: 20.06.1989
(51) Int. Cl.: C07C 45/67

(54) **Verfahren zur Herstellung von Ketonen**
Process for the preparation of ketones
Procédé pour la préparation de cétones

(30) Priorität: 30.06.1988 DE 3822070
(43) Veröffentlichungstag der Anmeldung: 03.01.1990
(73) Patentinhaber: BASF Aktiengesellschaft, 67063 Ludwigshafen (DE)
(72) Erfinder: Hoelderich, Wolfgang, Dr., D-6710 Frankenthal (DE); Merger, Franz, Dr., D-6710 Frankenthal (DE); Fischer, Rolf, Dr., D-6900 Heidelberg (DE)

(56) Entgegenhaltungen:
- EP-A- 0 162 387
- EP-A- 0 262 533
- DE-B- 1 253 694
- FR-A- 1 453 634
- US-A- 3 236 896
- US-A- 4 329 506
- US-A- 4 537 995

## Beschreibung

Die vorliegende Erfindung betrifft ein neues und verbessertes Verfahren zur Herstellung von Ketonen durch Isomerisierung von Aldehyden, die eine weitere funktionelle Gruppierung tragen, unter Verwendung von Zeolithen als Katalysatoren.

Ketone sind wegen ihrer vielseitigen Verwendungsmöglichkeiten gesuchte chemische Verbindungen. Man verwendet sie z.B. als Lösungsmittel in der Gummi-und Kunststoffindustrie, als Lösungsmittel bei chemischen Reaktionen, z.B. als Zwischenprodukte für Farbstoffe, Pflanzenschutzmittel und Pharmaprodukte und als Duftstoffe. Die Herstellung von Ketonen aus Aldehyden durch Isomerisierung ist wünschenswert, da Aldehyde z.B. über die Oxosynthese leicht zugänglich sind. Isomerisierungen dieser Art sind bekannt. Man führt sie z.B. an Katalysatoren aus Zinn-, Molybdän- und Kupfer-enthaltenden Mischoxiden (US-A-4 329 506) oder an Ceroxid auf Aluminiumoxid (US-A-3 466 334) durch. Von Nachteil bei diesen Verfahren ist, daß nur niedrige Selektivitäten erzielt werden und daß sich die besten Ergebnisse hinsichtlich Selektivität und Standzeit nur unter Zusatz von Wasserdampf erreichen lassen. Die schnelle Desaktivierung der Katalysatoren durch Koksabscheidung wirkt sich ebenfalls nachteilig aus. Man war deshalb bei der technischen Herstellung von unsymmetrisch substituierten Ketonen in der Regel auf die Kondensation unterschiedlicher organischer Säuren unter Decarboxylierung angewiesen, wie sie aus der DE-A-2 758 113 bekannt waren. Bei diesem Verfahren ist der Zwangsanfall von symmetrisch substituierten Ketonen und von Kohlendioxid von Nachteil.

Aus der US-A-4,537,995 ist ein Verfahren zur Isomerisierung von verzweigten Aldehyden zu Ketonen an Zeolithen bekannt, wobei die Aldehyde keine funktionelle Gruppe in α-Position zur Aldehydfunktion tragen.

Auch ist aus EP-A-162 387 bekannt, daß man Aldehyde an Zeolithen zu Ketonen isomerisieren kann. Doch tragen diese Aldehyde keine weitere funktionellen Gruppen neben der Aldehydfunktion.

Der vorliegenden Erfindung lag daher die Aufgabe zugrunde, den genannten Nachteilen abzuhelfen.

Demgemäß wurde ein neues und verbessertes Verfahren zur Herstellung von Ketonen aus Aldehyden der allgemeinen Formel I 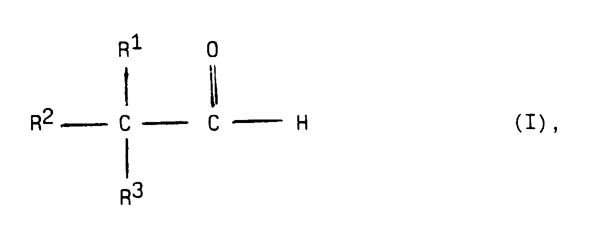
in der R¹ und R² unabhängig voneinander Wasserstoff, C₁- bis C₁₀-Alkyl, Aryl oder C₇- bis C₂₀-Aralkyl und R³ C₂- bis C₁₀-Alkenyl, C₈- bis C₂₀-Arylalkenyl, C₁- bis C₁₀-Hydroxyalkyl, C₂- bis C₁₀-Acyl, C₂- bis C₁₀-Acyloxy, C₂- bis C₁₀-Alkoxyalkyl, C₁- bis C₁₀-Alkoxy oder C₂- bis C₁₀-Alkenyloxy bedeuten, mit der Maßgabe, daß R¹ und R² nicht gleichzeitig für Wasserstoff stehen, wenn R³ für C₁- bis C₁₀-Alkoxy oder C₂- bis C₁₀-Alkenyloxy steht, gefunden, welches dadurch gekennzeichnet ist, daß man die Umsetzung in Gegenwart von Alumino-, Boro-, Eisen-, Beryllium-, Gallium-, Chrom-, Arsen-, Antimon- und Wismutsilikatzeolithode oder deren Gemische sowie Alumino-, Boro-, Gallium- und Eisengermanatzeolithe oder deren Gemische durchführt.

Die Ketone sind nach folgender Methode erhältlich:
Die Umsetzung erfolgt durch Kontakt eines Aldehyds, das eine, zwei oder drei unter den Reaktionsbedingungen inerte funktionelle Gruppen trägt, nach folgender Reaktionsgleichung: 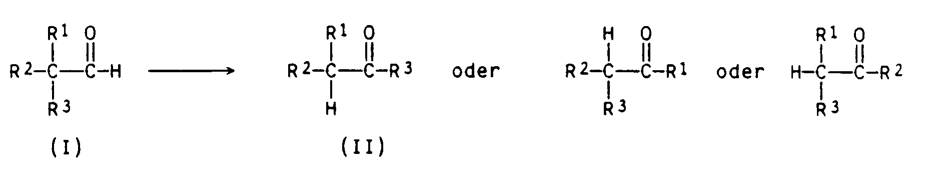
Die Reaktion kann sowohl in der Flüssigphase als auch in der diskontinuierlichen oder vorzugsweise kontinuierlichen Gasphase bei 50 bis 600°C und 0,01 bis 50 bar durchgeführt werden.

Die Flüssigphasenreaktion kann beispielsweise als Suspensions-, Riesel- oder Sumpfreaktion bei Temperaturen von 50 bis 200°C und Drücken von 0,5 bis 20 bar, vorzugsweise bei Temperaturen von 70 bis 170°C und Drücken von 1 bis 5 bar durchgeführt werden.

Die bevorzugte Gasphasenreaktion kann beispielsweise bei Temperaturen von 100 bis 600°C, vorzugsweise bei 150 bis 400°C und Drücken von 0,1 bis 50 bar, besonders bevorzugt bei 200 bis 300°C und Drücken von 0,5 bis 5 bar, gegebenenfalls in Gegenwart von Inertgasen wie Stickstoff oder Argon, in einigen Fällen auch Sauerstoff oder auch z.B. Wasserdampf, durchgeführt werden. Bei der Umsetzung in der Gasphase hält man vorteilhaft eine Katalysatorbelastung von 0,1 bis 20, insbesondere 0,5 bis 8 g Aldehyd je g Katalysator und Stunde ein (WHSV = g Einsatzgemisch/g Katalysator und Stunde). Die Gasphasenreaktion kann in einem Festbett oder in einem Wirbelbett ausgeführt werden.

Es empfiehlt sich schwerflüchtige oder feste Aldehyde in einem inerten Lösungsmittel gelöst zu verwenden, wobei pro Mol Aldehyd zwischen 100 und 500 ml, vorzugsweise 150 bis 350 ml eines inerten Lösungsmittels in aller Regel genügen.

Als inerte Lösungsmittel eignen sich Ether, besonders cyclische Ether wie Tetrahydrofuran; aliphatische Kohlenwasserstoffe wie n-Pentan, das Pentanisomerengemisch, n-Hexan, das Hexanisomerengemisch, Petrolether und Cyclohexan; aromatische Kohlenwasserstoffe wie Benzol, Toluol, die Xylole und deren Isomerengemisch oder Mischungen dieser Lösungsmittel.

Nach der Umsetzung werden die entstandenen Produkte durch übliche Verfahren, z.B. durch Destillation aus dem Reaktionsgemisch isoliert; nichtumgesetzte Ausgangsstoffe werden gegebenenfalls in die Umsetzung zurückgeführt.

Bei dieser Arbeitsweise werden gasförmige Reaktionsprodukte sofort in eine Trennung eingebracht und z.B. in einer Fraktionierkolonne in ihre Einzelkomponenten zerlegt. Eine weitere Ausführungsform besteht darin, daß man die Reaktionsausträge in einer wäßrigen Hydrogencarbonat-Lösung z.B. KHCO₃ oder NaHCO₃/Na₂SO₄ quencht.

Als funktionelle Gruppen eignen sich beispielsweise Alkenylgruppen, Hydroxyalkylgruppen, Acylgruppen, Arylalkenylgruppen, Acyloxygruppen, Alkoxyalkylgruppen, Alkoxygruppen und Alkenyloxygruppen, insbesondere diejenigen die für R³ nachfolgend aufgeführt werden. Die Aldehyde können ein, zwei oder drei dieser Gruppen tragen. Solche sind unter den Reaktionsbedingungen inert.

Bevorzugte Aldehyde sind diejenigen der allgemeinen Formel I.

Die Substituenten R¹ und R² in den Formeln I und II bedeuten Wasserstoff, unverzweigtes oder verzweigtes C₁- bis C₁₀-Alkyl, insbesondere unverzweigtes oder verzweigtes C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, tert.-Pentyl, neo-Pentyl, 1,2-Dimethylpropyl, n-Hexyl, iso-Hexyl, sec.-Hexyl, n-Heptyl, iso-Heptyl, n-Octyl und iso-Octyl, Aryl, z.B. Phenyl, C₇- bis C₂₀-Aralkyl, bevorzugt C₇- bis C₁₀-Aralkyl wie Benzyl und Phenethyl.

Der Substituent R³ in den Formeln I und II bedeutet C₂- bis C₁₀-Alkenyl, bevorzugt C₂- bis C₆-Alkenyl wie Vinyl, Allyl, Butenyl, Pentenyl, Hexenyl und deren Isomere, C₈- bis C₂₀-Arylalkenyl, bevorzugt C₈- bis C₁₂-Arylalkenyl wie 1-Phenylvinyl, 2-Phenylvinyl, 1-Phenylallyl, 2-Phenylallyl und 3-Phenylallyl, C₁- bis C₁₀-Hydroxyalkyl, bevorzugt C₁- bis C₆-Hydroxyalkyl wie Hydroxymethyl, 1-Hydroxyethyl, 2-Hydroxyethyl, 1-Hydroxypropyl, 2-Hydroxypropyl, 1-Hydroxybutyl, 2-Hydroxybutyl und 3-Hydroxybutyl, C₂- bis C₁₀-Acyl, bevorzugt C₂- bis C₆-Acyl wie Methylcarbonyl, Ethylcarbonyl, Propylcarbonyl, Butylcarbonyl, Pentylcarbonyl, Hexylcarbonyl und deren Isomere, C₂- bis C₁₀-Acyloxy, bevorzugt C₂- bis C₆-Acyloxy wie Acetyl, Propionyl, Butyryl, Valeroyl, Caproyl und deren Isomere, C₂- bis C₁₀-Alkoxyalkyl, bevorzugt C₂- bis C₆-Alkoxyalkyl wie Methoxymethyl, 1-Methoxyethyl, 2-Methoxyethyl, Ethoxymethyl, Propoxymethyl, Butoxymethyl, Pentoxymethyl, Methoxypropyl, Ethyloxypropyl und Ethoxybutyl, C₁- bis C₁₀-Alkoxy, bevorzugt C₁- bis C₈-Alkoxy wie Methoxy, Ethoxy, Propoxy, Butoxy, Pentoxy, Hexoxy, Heptoxy, Octoxy und deren Isomere, C₂- bis C₁₀-Alkenyloxy, bevorzugt C₂- bis C₆-Alkenyloxy wie Vinyloxy, Allyloxy, Propenyloxy, Butenyloxy, Pentenyloxy, Hexenyloxy und deren Isomere.

Für den Fall, daß R¹ und R² gleichzeitig für Wasserstoff stehen, ist C₁-bis C₁₀-Alkoxy und C₂- bis C₁₀-Alkenyloxy als Bedeutung für R³ ausgeschlossen.

Als Aldehyde seien beispielsweise 2-Methoxypropanal, 2-Acetoxypropanal, 4-Acetoxy-2-methyl-butanal, 2-Propioxypropanal, Isobutenyloxypivalaldehyd, Butoxypivalaldehyd, Isobutoxypivalaldehyd, Methoxypivalaldehyd, Isopropenylpivalaldehyd, Isobutyroxypivalaldehyd, Hydroxypivalaldehyd, 2,2-Dimethylpenten-4-al, 2-Methyl-2-phenyl-penten-4-al, 2,2-Dimethyl-5-methylenhexen-4-al und 2,2-Dimethyl-4-methyl-penten-4-al genannt. Die hier aufgeführten Verbindungen stellen eine Auswahl verwendbarer Komponenten für die erfindungsgemäße Isomerisierung dar und sollen die Anwendungsbreite des erfindungsgemäßen Verfahrens nicht einschränken. Die Aldehyde lassen sich nach bekannten Methoden z.B. durch Hydroformylierung von Olefinen herstellen.

Neben der Isomerisierung kann, insbesondere bei den mit Alkoxy- und Alkenyloxyresten substituierten Aldehyden gleichzeitig Eliminierung eines Alkohols auftreten, z.B. gemäß folgender Gleichung: 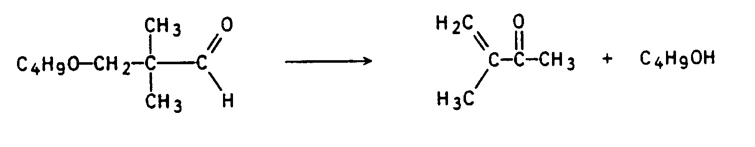
Als Katalysatoren für das erfindungsgemäße Verfahren werden acide zeolithische Katalysatoren eingesetzt. Zeolithe sind kristalline Aluminosilikate, die eine hochgeordnete Struktur mit einem starren dreidimensionalen Netzwerk von SiO₄- und AlO₄-Tetraedern besitzen, die durch gemeinsame Sauerstoffatome verbunden sind. Das Verhältnis der Si- und Al-Atome zu Sauerstoff beträgt 1:2 (siehe Ullmanns Encyclopädie d. techn. Chemie, 4. Auflage, Band 24, Seite 575 (1983)). Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Entsprechend ihrer Struktur werden Zeolithe in verschiedene Gruppen unterteilt (siehe Ullmanns Encyclopädie d. techn. Chemie, 4. Auflage, Band. 24, S. 575 (1983)). So bilden bei der Mordernit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder Y.

Für das erfindungsgemäße Verfahren in Betracht kommende Katalysatoren sind Zeolithe aus der Mordenit-Gruppe oder engporige Zeolithe vom Erionit- bzw. Chabasit-Typ oder Zeolithe vom Faujasit-Typ, z.B. Y-, X- oder L-Zeolithe. In diese Gruppe von Zeolithen gehören auch die sogenannten "ultrastabile" Zeolithe des Faujasittyps, d.h. dealuminierte Zeolithe. Verfahren zur Herstellung solcher Zeolithe sind beschrieben in "Catalysis by Zeolites", Band 5 aus "Studies in Surface Science and Catalysis" ed. B. Imelik et al. Elsevier Scientific Publishing Comp., 1980, 203 und "Crystal Structures of Ultra-stable Faujasites" Advances in Chemistry Series Nr. 101, American Chemical Society Washington, DC, S. 226 ff (1971) und in US-A-4 512 961.

Besonders vorteilhaft sind Zeolithe vom Pentasiltyp. Diese haben als Grundbaustein einen aus SiO₄-Tetraedern aufgebauten Fünfring gemeinsam. Sie sind durch ein hohes SiO₂/Al₂O₃-Verhältnis gekennzeichnet sowie durch Porengrößen, die zwischen denen der Zeolithe vom Typ A und denen vom Typ X oder Y liegen (vgl. Ullmanns Encyclopädie d. techn. Chem., 4. Aufl., Bd. 24, 1983).

Diese Zeolithe können unterschiedliche chemische Zusammensetzung aufweisen. Es handelt sich hierbei um Alumino-, Boro-, Eisen-, Beryllium-, Gallium-, Chrom-, Arsen-, Antimon- und Wismutsilikatzeolithe oder deren Gemische sowie Alumino-, Boro-, Gallium- und Eisengermanatzeolithe oder deren Gemische. Insbesondere eignet sich die Alumino-, Boro- und Eisensilikatzeolithe des Pentasiltyps für das erfindungsgemäße Verfahren. Der Aluminosilikatzeolith wird z.B. aus einer Aluminiumverbindung, vorzugsweise Al(OH)₃ oder Al₂(SO₄)₃ und einer Siliciumkomponente, vorzugsweise hochdispersem Siliciumdioxid in wäßriger Aminlösung, insbesondere in Polyaminen wie 1,6-Hexandiamin- oder 1,3-Propandiamin- oder Triethylentetramin-Lösung mit oder insbesondere ohne Alkali- oder Erdalkalizusatz bei 100 bis 220°C unter autogenem Druck hergestellt. Auch gehören hierzu die isotaktischen Zeolithe nach EP-A-34 727 und EP-A-46 504. Die erhaltenen Aluminosilikatzeolithe enthalten je nach Wahl der Einsatzstoffmengen ein SiO₂/Al₂O₃-Verhältnis von 10 bis 40.000. Auch lassen sich derartige Aluminosilikatzeolithe in etherischem Medium wie Diethylenglykoldimethylether, in alkoholischem Medium wie Methanol bzw. 1,4-Butandiol oder in Wasser synthetisieren.

Der Borosilikatzeolith wird z.B. bei 90 bis 200°C unter autogenem Druck synthetisiert, indem man eine Borverbindung z.B. H₃BO₃, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Auch gehören hierzu die isotaktischen Zeolithe nach EP-A-34 727 und EP-A-46 504. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhält man z.B. aus einer Eisenverbindung, vorzugsweise Fe₂(SO₄)₃ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220°C unter autogenem Druck.

Zu den verwendbaren siliciumreichen Zeolithen (SiO₂/Al₂o₃≧10) gehören auch die sog. ZSM-Typen, Ferrierit, Nu-1 und Silicalit®, ein Molekularsieb, ein sog. Silica Polymorph.

Die so hergestellten Alumino-, Boro- und Eisensilikatzeolithe können nach ihrer Isolierung, Trocknung bei 100 bis 160°C, vorzugsweise 110°C und Calcinierung bei 450 bis 550°C, vorzugsweise 500°C, mit einem Bindemittel im Verhältnis 90:10 bis 40:60 Gew.% zu Strängen oder Tabletten verformt werden. Als Bindemittel eignen sich diverse Aluminiumoxide, bevorzugt Boehmit, amorphe Aluminosilikate mit einem SiO₂/Al₂O₃-Verhältnis von 25:75 bis 90:5, bevorzugt 75:25, Siliciumdioxid, bevorzugt hochdisperses SiO₂, Gemische aus hochdispersem SiO₂ und hochdispersem Al₂O₃, TiO₂, ZrO₂ sowie Ton. Nach der Verformung werden die Extrudate oder Preßlinge bei 110°C/16 h getrocknet und bei 500°C/16 h calciniert.

Man erhält auch vorteilhafte Katalysatoren, wenn der isolierte Alumino- bzw. Borosilikatzeolith direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eventuell eine durch Koksabscheidung bedingte Desaktivierung eintritt, empfiehlt es sich, die Zeolithe durch Abbrennen der Koksablagerung mit Luft oder mit einem Luft/N₂-Gemisch bei 400 bis 550°C, bevorzugt 500°C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, kann es vorteilhaft sein, die Zeolithe zu modifizieren. Eine geeignete Modifizierung der Katalysatoren besteht z.B. darin, daß man den unverformten oder veformten Zeolithen mit Metallsalzen durch einen Ionenaustausch oder durch Imprägnierung dotiert. Als Metalle werden Alkalimetalle wie Li, Cs, K, Erdalkalimetalle wie Mg, Ca, Sr, Metalle der 3., 4. und 5. Hauptgruppe wie Al, Ga, Ge, Sn, Pb, Bi, Übergangsmetalle der 4. bis 8. Nebengruppe wie Ti, Zr, V, Nb, Cr, Mo, W, Mn, Re, Fe, Ru, Os, Co, Rh, Sr, Ni, Pd, Pt, Übergangsmetalle der 1. und 2. Nebengruppe wie Cu, Ag, Zn, Seltene Erdmetalle wie La, Ce, Pr, Nd, Fr, Yb und U eingesetzt.

Zweckmäßigerweise führt man die Dotierung so durch, daß man z.B. den verformten Zeolithen in einem Steigrohr vorlegt und bei 20 bis 100°C z.B. eine wäßrige oder ammoniakalische Lösung eines Halogenids oder eines Nitrats der voranbeschriebenen Metalle überleitet. Ein derartiger Ionenaustausch kann z.B. an der Wasserstoff-, Ammonium- und Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man Cu(NO₃)₂ x 3 H₂O oder Ni(NO₃)₂ x 6 H₂O oder Ce(NO₃)₃ x 6 H₂O oder La(NO₃)₂ x 6 H₂O oder Cs₂CO₃ in Wasser löst. Mit dieser Lösung wird der verformte oder unverformte Zeolith eine gewisse Zeit, ca. 30 Minuten, getränkt. Die eventuell überstehende Lösung wird am Rotationsverdampfer von Wasser befreit. Danach wird der getränkte Zeolith bei ca. 150°C getrocknet und bei ca. 550°C calciniert. Dieser Tränkvorgang kann mehrmals hintereinander vorgenommen werden, um den gewünschten Metallgehalt einzustellen.

Auch ist es möglich, z.B. eine wäßrige Ni(CO₃)₂-Lösung oder ammoniakalische Pd(NO₃)₂-Lösung herzustellen und darin den reinen pulverförmigen Zeolithen bei 40 bis 100°C unter Rühren ca. 24 h aufzuschlämmen. Nach Abfiltrieren, Trocknen bei ca. 150°C und Calcinierung bei ca. 500°C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber z.B. eine wäßrige Ni(NO₃)₂-Lösung oder ammoniakalische Pd(NO₃)₂-Lösung bei leicht erhöhter Temperatur zwischen 30 und 80°C im Kreislauf 15 bis 20 h leitet. Danach wird mit Wasser ausgewaschen, bei ca. 150°C getrocknet und bei ca. 550°C calciniert. Bei manchen metalldotierten Zeolithen z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft z.B. so vor, daß man Zeolithe in Pulverform mit 1 n Phosphorsäure 1 Stunde bei 80°C behandelt. Nach der Behandlung wird mit Wasser gewaschen, bei 110°C/16 Stunden getrocknet und bei 500°C/20 Stunden calciniert. Nach einer anderen Arbeitsweise behandelt man Zeolithe vor oder nach ihrer Verformung mit Bindemitteln, z.B. 1 bis 3 Stunden bei Temperaturen von 60 bis 80°C mit einer 3 bis 25 gew.%igen, insbesondere 12 bis 20 gew.%igen wäßrigen Salzsäure. Anschließend wird der so behandelte Zeolith mit Wasser gewaschen, getrocknet und bei 400°C bis 500°C calciniert.

Eine besondere Ausführungsform für die Säurebehandlung besteht darin, daß man das zeolithische Material vor seiner Formung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von im allgemeinen 0,5 bis 5, vorzugsweise 1 bis 3 Stunden. Nach Isolierung, z.B. durch Abfiltrieren und Auswaschen, des zeolithischen Materials wird dieses zweckmäßig, z.B. bei Temperaturen von 100 bis 160°C, getrocknet und bei Temperaturen von im allgemeinen 450 bis 600°C calciniert. Gemäß einer weiteren bevorzugten Ausführungsform für die Säurebehandlung wird das zeolithische Material nach einer Verformung mit Bindemittel bei erhöhter Temperatur, zweckmäßig bei Temperaturen von 50 bis 90°C, vorzugsweise 60 bis 80°C, über einen Zeitraum von 0,5 bis 5, vorzugsweise mit 12 bis 20 gew.%iger Salzsäure, behandelt. Anschließend wird das zeolithische Material im allgemeinen ausgewaschen und zweckmäßig, z.B. bei Temperaturen von 100 bis 160°C, getrocknet und bei Temperaturen von im allgemeinen 450 bis 600°C calciniert. Einer HF-Behandlung kann sich auch eine HCl-Behandlung anschließen.

Nach einer anderen Arbeitsweise lassen sich Zeolithe durch Aufbringung von Phosphorverbindungen, wie Trimethoxiphosphat, Trimethoxiphosphin, primärem, sekundärem oder tertiärem Natriumphosphat modifizieren. Hierbei werden die Zeolithe in Strang-, Tabletten- oder Wirbelgut-Form mit wäßriger H₃PO₄-Lösung getränkt, bei 110°C getrocknet und bei 500°C calciniert.

Die hier beschriebenen Katalysatoren können wahlweise als 2- bis 4-mm-Stränge oder als Tabletten mit 3 bis 5 mm Durchmesser als Splitt mit Teilchengrößen von 0,1 bis 0,5 mm oder als Wirbelkontakt eingesetzt werden.

Die folgenden Beispiele veranschaulichen die Erfindung:
Die Reaktionen in der Gasphase wurden unter isothermen Bedingungen in einem Rohrreaktor (Wendel, 0,6 cm Innendurchmesser, 90 cm Länge) mindestens 6 Stunden lang durchgeführt. Die Reaktionsprodukte wurden durch übliche Methoden abgetrennt und charakterisiert. Die quantitative Bestimmung der Reaktionsprodukte und der Ausgangsprodukte erfolgte gaschromatographisch.

### Herstellung der Katalysatoren

### Katalysator A

Der Borosilikatzeolith des Pentasil-Typs wird in einer hydrothermalen Synthese aus 640 g hochdispersem SiO₂, 122 g H₃BO₃, 8000 g einer wäßrigen 1,6-Hexandiamin-Lösung (Mischung 50:50 Gew.%) bei 170°C unter autogenem Druck in einem Rührautoklaven hergestellt. Nach Abfiltrieren und Auswaschen wird das kristalline Reaktionsprodukt bei 100°C/24 Stunden getrocknet und bei 500°C/24 h calciniert. Dieser Borosilikatzeolith setzt sich zusammen aus 94,2 Gew.% SiO₂ und 2,3 Gew.% B₂O₃.

Katalysator A wird erhalten, indem man den Borosilikatzeolith mit Boehmit im Gewichtsverhältnis 60:40 zu 2-mm-Strängen verformt, bei 110°C/16 h trocknet und bei 500°C/24 h calciniert.

### Katalysator B

Ein Aluminosilikatzeolith vom Pentasil-Typ wurde unter hydrothermalen Bedingungen bei autogenem Druck und 150°C aus 65 g hochdispersem SiO₂, 20,3 g Al₂(SO₄)₃ x 18 H₂O in 1 kg einer wäßrigen 1,6-Hexandiamin-Lösung (Mischung 50:50 Gew.%) in einem Rührautoklaven hergestellt. Nach Abfiltrieren und Auswaschen wurde das kristalline Reaktionsprodukt bei 110°C/24 Stunden getrocknet und bei 500°C/24 Stunden calciniert. Dieser Aluminosilikatzeolith enthielt 91,6 Gew.% SiO₂ und 4,6 Gew.% Al₂O₃.

Der Katalysator wurde mit Boehmit im Gewichtsverhältnis 60:40 zu 2-mm-Strängen verformt, bei 110°C/16 Stunden getrocknet und bei 500°C/24 Stunden calciniert.

### Katalysator C

Der Eisensilikatzeolith des Pentasil-Typs wurde unter hydrothermalen Bedingungen bei autogenem Druck und 165°C aus 273 g Wasserglas, gelöst in 253 g einer wäßrigen 1,6-Hexandiamin-Lösung (Mischung 50:50 Gew.%) und 31 g Eisensulfat, gelöst ind 21 g 96 %iger Schwefelsäure und 425 g Wasser in einem Rührautoklaven während 4 Tagen synthetisiert. Der Zeolith wurde abfiltriert, ausgewaschen, bei 100°C/24 Stunden getrocknet und bei 500°C/24 h calciniert. Man erhielt einen Eisensilikatzeolithen mit einem SiO₂/Fe₂O₃-Verhältnis von 17,7 und einen Na₂O-Gehalt von 1,2 Gew.%. Der Katalysator wurde mit einem Verformungshilfsmittel zu 2,5-mm-Strängen verstrangt, bei 110°C/16 Stunden getrocknet und bei 500°C/24 Stunden calciniert.

### Katalysator D

Der Katalysator wurde in einer hydrothermalen Synthese aus 64 g SiO₂ (hochdisperse Kieselsäure), 12,2 g H₃BO₃, 800 g einer wäßrigen Hexandiamin-Lösung (Mischung 50:50 Gew.%) bei 170°C unter autogenem Druck in einem Rührautoklaven hergestellt. Nach Abfiltrieren und Auswaschen wurde das kristalline Reaktionsprodukt bei 100°C/24 Stunden getrocknet und bei 500°C/24 h calciniert. Es wurde ein Borosilikatzeolith vom Pentasiltyp erhalten, der 94,2 Gew.% SiO₂ und 2,32 Gew.% B₂O₃ enthielt. Aus diesem Zeolithen wurden mit einem Verformungshilfsmittel 2-mm-Stränge hergestellt, die bei 100°C getrocknet und bei 500°C/24 Stunden calciniert.

### Katalysator E

Der Katalysator wurde aus dem Borosilkatzeolithen, dessen Synthese bei Katalysator D beschrieben ist, hergestellt. Dabei wurde das Zeolithpulver mit hochdispersem SiO₂, das 0,3 bis 1,3 Gew.% hochdisperses Al₂O₃ enthielt, im Gewichtsverhältnis 60:40 zu 2-mm-Strängen verstrangt. Die Stränge wurden bei 110°C/16 Stunden getrocknet und bei 500°C/16 Stunden calciniert.

### Katalysator F

Der Borosilikatzeolith von Katalysator D wurde mit Boehmit im Gewichtsverhältnis 60:40 zu 2-mm-Strängen verformt. Die bei 110°C/16 Stunden getrocknet und bei 500°C/16 Stunden calcinierten Stränge wurden mit einer wäßrigen Lösung Cernitrat 30 min getränkt. Das Restwasser wurde am Rotationsverdampfer abgesaugt, die Stränge getrocknet und calciniert. Der Ce-Gehalt betrug 6,8 Gew.%.

Die mit diesen Katalysatoren erzielten Versuchsergebnisse sowie die Versuchsbedingungen sind in nachfolgenden Beispielen angegeben.

### Beispiel 1 bis 3

2-Methoxypropionaldehyd wird im vorangehend beschriebenen Reaktor unter isothermen Bedingungen an Katalysator D mit WHSV = 2,5 h⁻¹ zu Methoxyaceton isomerisiert. Es werden folgende Umsätze und Selektivitäten (Berechnung basiert auf GC-Flächen-%) erzielt.

| | |
|---|---|
| Beispiel 1 bei 350°C: 62,7 % Umsatz | 87,8 % Selektivität |
| Beispiel 2 bei 400°C: 71,6 % Umsatz | 89,0 % Selektivität |
| Beispiel 3 bei 450°C: 81,9 % Umsatz | 88,4 % Selektivität |

### Beispiel 4 bis 9

2-Acetoxypropionaldehyd wird zu Acetoxyaceton in obiger Apparatur umgesetzt. Die Versuchsergebnisse sind in nachstehender Tabelle aufgelistet.

| Beispiel | Katalysator | Temperatur | WHSV | Umsatz | Selektivität |
|---|---|---|---|---|---|
| 4 | A | 250°C | 2 h⁻¹ | 41,5 | 93,7 |
| 5 | A | 350°C | 2 h⁻¹ | 75,9 | 80,6 |
| 6 | A | 400°C | 2 h⁻¹ | 80,7 | 63,8 |
| 7 | E | 350°C | 3 h⁻¹ | 61,6 | 77,6 |
| 8 | D | 300°C | 2,5 h⁻¹ | 74,6 | 71,6 |
| 9 | B | 300°C | 2 2,5⁻¹ | 54,6 | 83,3 |

### Beispiel 10

4-Acetoxy-2-methylbutanal wird zu 5-Acetoxy-pentan-2-on in obiger Apparatur umgesetzt. Bei 150°C und WHSV = 2,5 h⁻¹ erhält man 38 % Selektivität bei 90,5 % Umsatz.

### Beispiel 11

Isobutenyloxypivalaldehyd wird an Katalysator A bei 300°C und WHSV = 2 h⁻¹ zu 4-Isobutenyloxy-3-methyl-butan-2-on umgesetzt. Der Umsatz beträgt 50,8 % und die Selektivität 69,4 %.

### Beispiel 12 und 13

2,2-Dimethyl-penten-4-al wird in obiger Apparatur zu 3-Methyl-hex-5-en-2-on (Methylwanderung) und Isopropyl-n-propenyl-keton (Allylwanderung) umgesetzt. An Katalysator D (Beispiel 12) werden bei 300°C und WHSV = 2 h⁻¹ 69,2 % Umsatz, 10,5 % Selektivität für 3-Methyl-hex-5-en-2-on und 57,5 % Selektivität für Isopropyl-n-propenyl-keton erzielt. An Katalysator C (Beispiel 13) werden unter gleichen Reaktionsbedingungen 41 % Umsatz, 20,2 % Selektivität für 3-Methyl-hex-5-en-2-on und 45,1 % Selektivität für Isopropyl-n-propenyl-keton erhalten. Man erkennt, daß man durch Wahl des zeolithischen Katalysators bevorzugt Allyl- oder Methylwanderung bewirken kann.

### Beispiel 14

2-Methyl-2-phenyl-pent-4-enal wird in obiger Apparatur bei 400°C und WHSV = 3 h⁻¹ an Katalysator D zu 3-Phenyl-hex-5-en-2-on (Methylwanderung) und 2-Phenyl-hex-5-en-3-on (Allylwanderung) im Verhältnis 4,5:1 umgesetzt. Der Umsatz beträgt 47,8 % und die Gesamtselektivität an Wertprodukten 78,5 %.

### Beispiel 15

n-Butoxypivalaldehyd wird an Katalysator A bei 400°C und mit WHSV = 2 h⁻¹ umgesetzt. Hierbei finden sowohl eine Wanderung der Methylgruppe als auch eine Eliminierung von n-Butanol statt und es entsteht Methylisopropenylketon mit einem Umsatz von 100 % und einer Selektivität von 59 %.

### Beispiel 16 und 17

Die Reaktion aus Beispiel 15 wird unter gleichen Bedingungen am Katalysator C (Beispiel 16) und Katalysator F (Beispiel 17) durchgeführt. An Katalysator C werden 98,4 % Umsatz und 65,6 % Selektivität von Methylisopropenylketon und an Katalysator F 96,4 % Umsatz und 45,9 % Selektivität von Methylisopropenylketon erhalten.

## Patentansprüche

1. Verfahren zur Herstellung von Ketonen aus Aldehyden der allgemeinen Formel I 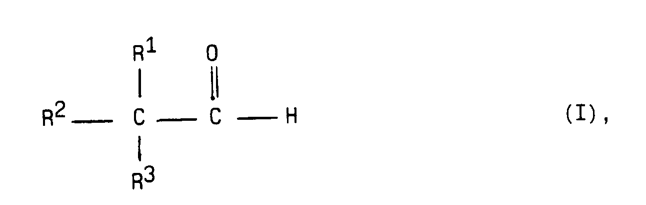 in der R¹ und R² unabhängig voneinander Wasserstoff, C₁- bis C₁₀-Alkyl, Aryl oder C₇- bis C₂₀-Aralkyl und R³ C₂- bis C₁₀-Alkenyl, C₈- bis C₂₀-Arylalkenyl, C₁- bis C₁₀-Hydroxyalkyl, C₂- bis C₁₀-Acyl, C₂- bis C₁₀-Acyloxy, C₂- bis C₁₀-Alkoxyalkyl, C₁- bis C₁₀-Alkoxy oder C₂- bis C₁₀-Alkenoyloxy bedeuten, mit der Maßgabe, daß R¹ und R² nicht gleichzeitig für Wasserstoff stehen, wenn R³ für C₁- bis C₁₀-Alkoxy oder C₂- bis C₁₀-Alkenyloxy steht, dadurch gekennzeichnet, daß man die Umsetzung in Gegenwart von Alumino-, Boro-, Eisen-, Beryllium-, Gallium-, Chrom-, Arsen-, Antimon- und Wismutsilikatzeolithode oder deren Gemische sowie Alumino-, Boro-, Gallium- und Eisengermanatzeolithe oder deren Gemische durchführt.

2. Verfahren zur Herstellung von Ketonen aus Aldehyden I nach Anspruch 1, dadurch gekennzeichnet, daß man Alumino-, Boro - und Eisensilikatzeolithe des Pentasiltyps verwendet.

## Claims

1. A process for preparing a ketone from an aldehyde of the general formula I 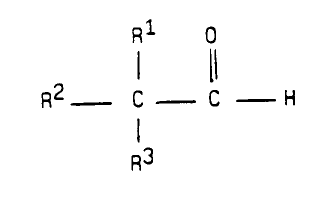 where R¹ and R² are each independently of the other hydrogen, C₁-C₁₀-alkyl, aryl or C₇-C₂₀-aralkyl and R³ is C₂-C₁₀-alkenyl, C₈-C₂₀-arylalkenyl, C₁-C₁₀-hydroxyalkyl, C₂-C₁₀-acyl, C₂-C₁₀-acyloxy, C₂-C₁₀-alkoxyalkyl, C₁-C₁₀-alkoxy or C₂-C₁₀-alkenyloxy, with the proviso that R¹ and R² are not both hydrogen when R³ is C₁-C₁₀-alkoxy or C₂-C₁₀-alkenyloxy, which comprises carrying out the reaction in the presence of an aluminosilicate, borosilicate, iron silicate, beryllium silicate, gallium silicate, chromium silicate, arsenic silicate, antimony silicate or bismuth silicate zeolite or a mixture thereof or an aluminogermanate, borogermanate, gallium germanate or iron germanate zeolite or a mixture thereof.

2. A process for preparing a ketone from an aldehyde I as claimed in claim 1, wherein an aluminosilicate, borosilicate or iron silicate zeolite of the pentasil type is used.

## Revendications

1. Procédé de préparation de cétones à partir d'aldéhydes de formule générale I 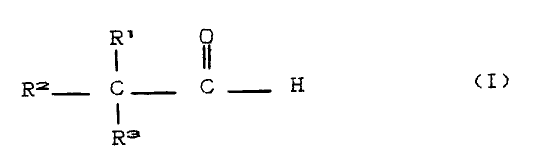 dans laquelle R¹ et R² indépendamment l'un de l'autre représentent un atome d'hydrogène, un groupe alkyle en C₁ à C₁₀, aryle ou aralkyle en C₇ à C₂₀ et R³ un groupe alcényle en C₂ à C₁₀, arylalcényle en C₈ à C₂₀, hydroxyalkyle en C₁ à C₁₀, acyle en C₂ à C₁₀, acyloxy en C₂ à C₁₀, alcoxyalkyle en C₂ à C₁₀, alcoxy en C₁ à C₁₀ ou alcényloxy en C₂ à C₁₀, à condition que R¹ et R² ne soient pas simultanément un atome d'hydrogène lorsque R³ est un groupe alcoxy en C₁ à C₁₀ ou alcényloxy en C₂ à C₁₀, caractérisé en ce que l'on effectue la réaction en présence de zéolites d'alumino-, boro-silicate, silicate de fer, beryllium, gallium, chrome, arsenic, antimoine ou bismuth, ou de leurs mélanges, ou en présence de zéolites d'alumino-, boro-germanate, germanate de gallium ou de fer, ou de leurs mélanges.

2. Procédé de préparation de cétones à partir d'aldehydes I selon la revendication 1, caractérisé en ce que l'on utilise des zéolites d'alumino-, boro-silicate, ou silicate de fer du type pentasil.
